(19) Europäisches Patentamt
European Patent Office
Office européen des brevets

(11) **EP 4 621 801 A1**

(12) **EUROPEAN PATENT APPLICATION**

(43) Date of publication:
24.09.2025 Bulletin 2025/39

(21) Application number: 25164011.6

(22) Date of filing: 17.03.2025

(51) International Patent Classification (IPC):
**G16H 50/20** (2018.01) **A61B 5/00** (2006.01)

(52) Cooperative Patent Classification (CPC):
**G16H 50/20; A61B 5/02416; A61B 5/7267**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 18.03.2024 IN 202421020145

(71) Applicant: **Tata Consultancy Services Limited
Maharashtra (IN)**

(72) Inventors:
• **CHATTERJEE, Subhasri
700135 Kolkata, West Bengal (IN)**

• **PAL, Rajat Subhra
700135 Kolkata, West Bengal (IN)**
• **VASUDEVAN, Smita Elayath
682042 Kochi, Kerala (IN)**
• **RAJADHYAKSHA, Ninad Yeshwant
400021 Mumbai, Maharashtra (IN)**
• **KUMAR, Shashank
700135 Kolkata, West Bengal (IN)**
• **GHOSE, Avik
700135 Kolkata, West Bengal (IN)**

(74) Representative: **Goddar, Heinz J.
Boehmert & Boehmert
Anwaltspartnerschaft mbB
Pettenkoferstrasse 22
80336 München (DE)**

(54) **METHODS AND SYSTEMS FOR FLUID RETENTION ANALYSIS IN CHRONIC KIDNEY CARE**

(57) The disclosure relates generally to methods and systems for fluid retention analysis in Chronic Kidney Care. Conventional techniques for detecting the CKD in the subject lack with efficiency and accuracy since most of the ailments are silent and variant in nature pinpointing a universal cause or biomarker is difficult. The methods and systems of the present disclosure propose an in-silico model-based approach to detect CKD in the subject. In the first stage, a standard PPG waveform is simulated using the physicsbased model. In the second stage, a Generative Adversarial Network (GAN) model is trained using the simulated PPG data and the reference experimental PPG data. In the third and the last stage, the discriminator model of the trained GAN model is employed to evaluate and analyze the test dataset of the subject whose CKD is to be detected.

FIG. 4

**Description**

CROSS-REFERENCE TO RELATED APPLICATIONS AND PRIORITY

The present application claims priority to Indian application no. 202421020145, filed on March 18, 2024.

TECHNICAL FIELD

The disclosure herein generally relates to Chronic Kidney Disease (CKD), and, more particularly, to methods and systems for fluid retention analysis in Chronic Kidney Care using Generative Artificial Intelligence (AI) aided by Physics based modelling.

BACKGROUND

As per the World Health Organization projects CKD is predicted to be the 5th most common chronic disease in 2040. Causes of CKD are multi-factorial and diverse, but early-stage symptoms are often few and silent. Progression rates are highly variable, but patients encounter both an increased risk for end-stage kidney disease (ESKD) as well as increased cardiovascular risk. End-stage kidney disease incidence is generally low, but every single case carries a significant burden of illness and healthcare costs, making prevention by early intervention both desirable and worthwhile.

Major symptoms of the kidney failure include but are not limited to itchy skin or rashes, muscle cramps, feeling sick to your stomach or throwing up, not feeling hungry than normal, swelling in your feet and ankles, urinating (peeing) more or less than normal, foamy, frothy, or bubbly-looking urine, trouble catching your breath, and trouble falling or staying asleep. Since most of the ailments are silent and variant in nature pinpointing a universal cause or biomarker is difficult. Hence, the conventional techniques for detecting the CKD in the subject lack with efficiency and accuracy. Moreover, getting patient health data brings privacy and security issues. Cost is a big factor for organizations doing the research, development, and implementation of such digital biomarkers as well as for patients who need to procure these devices.

SUMMARY

Embodiments of the present disclosure present technological improvements as solutions to one or more of the above-mentioned technical problems recognized by the inventors in conventional systems.

In an aspect, a processor-implemented method for fluid retention analysis in Chronic Kidney Care is provided. The method including the steps of: receiving (i) a data associated with one or more Photoplethysmography (PPG) based biomarkers, (ii) a data associated with a varying blood volume between a systole and a diastole, and (iii) one or more reference PPG signals data, wherein each of the one or more reference PPG signals is recorded for one more predefined bio-parameters; instantiating a physics-based PPG simulation model with (i) the data associated with the one or more Photoplethysmography (PPG) based biomarkers, and (ii) the data associated with the varying blood volume between the systole and the diastole, to obtain a pulsatile PPG model; generating one or more pulsatile PPG signals, using the pulsatile PPG model; training a PlethAugment-based conditional generative adversarial network (CGAN) model comprising a generator and a discriminator, with (i) the one or more pulsatile PPG signals and (ii) the one or more reference PPG signals, to obtain a trained CKD detection model, wherein the generator and the discriminator are trained in tandem, and wherein the training comprises: (a) passing (i) each of the one or more pulsatile PPG signals and (ii) each of the one or more reference PPG signals, to the generator, to obtain (i) one or more encoded pulsatile PPG signals and (ii) one or more encoded reference PPG signals; (b) passing (i) an encoded pulsatile PPG signal of the one or more encoded pulsatile PPG signals and (ii) the one or more encoded reference PPG signals, at each iteration, to the discriminator, to determine a value of a loss function of the PlethAugment-based CGAN model; (c) backpropagating one or more network weights of the PlethAugment-based CGAN model, based on the value of the loss function; and (d) repeating the steps (b) through (c) for each of the one or more encoded pulsatile PPG signals, to obtain the trained CKD detection model; receiving an input PPG signal of a subject whose CKD is to be detected; and passing the input PPG signal of the subject to the trained CKD detection model, to detect a presence or an absence of the CKD in the subject.

In another aspect, a system for fluid retention analysis in Chronic Kidney Care is provided. The system includes: a memory storing instructions; one or more Input/Output (I/O) interfaces; and one or more hardware processors coupled to the memory via the one or more I/O interfaces, wherein the one or more hardware processors are configured by the instructions to: receive (i) a data associated with one or more Photoplethysmography (PPG) based biomarkers, (ii) a data associated with a varying blood volume between a systole and a diastole, and (iii) one or more reference PPG signals data, wherein each of the one or more reference PPG signals is recorded for one more predefined bio-parameters; instantiate a physics-based PPG simulation model with (i) the data associated with the one or more Photoplethysmography (PPG) based biomarkers, and (ii) the data associated with the varying blood volume between the systole and the diastole, to obtain a pulsatile PPG model; generate one or more pulsatile PPG signals, using the pulsatile PPG model; train a PlethAugment-based conditional generative adversarial network (CGAN) model comprising a generator and a discriminator, with (i) the one or more pulsatile PPG signals and (ii) the one or more reference PPG signals, to obtain a trained CKD detection model, wherein the generator and the discriminator are trained in tandem, and wherein the training comprises: (a) passing (i) each of the one or more pulsatile PPG signals and (ii) each of the one or more reference PPG signals, to the generator, to obtain (i) one or more encoded pulsatile PPG signals and (ii) one or more encoded reference PPG signals; (b) passing (i) an encoded pulsatile PPG signal of the one or more encoded pulsatile PPG signals and (ii) the one or more encoded reference PPG signals, at each iteration, to the discriminator, to determine a value of a loss function of the PlethAugment-based CGAN model; (c) backpropagating one or more network weights of the PlethAugment-based CGAN model, based on the value of the loss function; and (d) repeating the steps (b) through (c) for each of the one or more encoded pulsatile PPG signals, to obtain the trained CKD detection model; receive an input PPG signal of a subject whose CKD is to be detected; and pass the input PPG signal of the subject to the trained CKD detection model, to detect a presence or an absence of the CKD in the subject.

In yet another aspect, there are provided one or more non-transitory machine-readable information storage mediums comprising one or more instructions which when executed by one or more hardware processors cause: receiving (i) a data associated with one or more Photoplethysmography (PPG) based biomarkers, (ii) a data associated with a varying blood volume between a systole and a diastole, and (iii) one or more reference PPG signals data, wherein each of the one or more reference PPG signals is recorded for one more predefined bio-parameters; instantiating a physics-based PPG simulation model with (i) the data associated with the one or more Photoplethysmography (PPG) based biomarkers, and (ii) the data associated with the varying blood volume between the systole and the diastole, to obtain a pulsatile PPG model; generating one or more pulsatile PPG signals, using the pulsatile PPG model; training a PlethAugment-based conditional generative adversarial network (CGAN) model comprising a generator and a discriminator, with (i) the one or more pulsatile PPG signals and (ii) the one or more reference PPG signals, to obtain a trained CKD detection model, wherein the generator and the discriminator are trained in tandem, and wherein the training comprises: (a) passing (i) each of the one or more pulsatile PPG signals and (ii) each of the one or more reference PPG signals, to the generator, to obtain (i) one or more encoded pulsatile PPG signals and (ii) one or more encoded reference PPG signals; (b) passing (i) an encoded pulsatile PPG signal of the one or more encoded pulsatile PPG signals and (ii) the one or more encoded reference PPG signals, at each iteration, to the discriminator, to determine a value of a loss function of the PlethAugment-based CGAN model; (c) backpropagating one or more network weights of the PlethAugment-based CGAN model, based on the value of the loss function; and (d) repeating the steps (b) through (c) for each of the one or more encoded pulsatile PPG signals, to obtain the trained CKD detection model; receiving an input PPG signal of a subject whose CKD is to be detected; and passing the input PPG signal of the subject to the trained CKD detection model, to detect a presence or an absence of the CKD in the subject.

In an embodiment, the generator is an auto-encoder, and the discriminator is a time-series Convolutional Neural Network-Long Short-Term Memory (CNN-LSTM) model.

In an embodiment, the loss function of the PlethAugment-based CGAN model is a sum of a generator loss and a discriminator loss, and wherein the generator loss is defined as a function of reconstruction loss, and the discriminator loss is defined as function of Binary Cross-Entropy loss.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is an exemplary block diagram of a system for fluid retention analysis in Chronic Kidney Care, in accordance with some embodiments of the present disclosure.
FIGS. 2A-2B illustrate exemplary flow diagrams of a processor-implemented method for fluid retention analysis in Chronic Kidney Care, using the system of FIG. 1, in accordance with some embodiments of the present disclosure.
FIG. 3 shows an exemplary physics-based PPG simulation model, in accordance with some embodiments of the present disclosure.
FIG. 4 is a flowchart showing the steps for obtaining the pulsatile PPG model by instantiating the physics-based PPG simulation model, in accordance with some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

There are various recommended approaches for creating a digital biomarker that can be utilized for chronic kidney disease (CKD) detection. Some of them include Omics-based approach, Genetic markers, use of digital biomarkers, and a combination of biomarkers. The Omics-based approach uses technologies such as genomics, transcriptomics, proteomics, and metabolomics to identify potential biomarkers associated with CKD. These technologies allow for a comprehensive analysis of different molecular components and pathways involved in CKD. For example, transcriptomic analysis can identify biomarkers that indicate the risk of disease progression, while proteomic analysis can help identify kidney-derived proteins that could serve as biomarkers of renal dysfunction. By combining data from multiple omics approaches, we can gain a better understanding of CKD and identify potential biomarkers.

Some of genetic markers, such as genes and genetic variants, have been linked to the risk or early detection of CKD. For instance, certain genetic variants in the APOL1 gene are strongly associated with non-diabetic CKD and can be used for risk prediction. Genetic testing for these variants can help identify individuals at high risk for CKD development and progression. Additionally, studying genetic markers in ethnic minorities, who may have a higher genetic risk, can provide valuable insights for early intervention and disease management.

Biomedical signals such as Photoplethysmography (PPG) signals can be used to identify digital biomarkers which act as proxy to fluid retention since PPG is a volumetric measurement. A deep learning model will be used to discriminate between normal and abnormally high fluid levels in the tissue, which is indicative of CKD.

Since CKD is a complex disease with multiple factors involved, relying on a single biomarker may not be enough for accurate diagnosis and risk assessment. Therefore, a panel of biomarkers that includes genetic markers, proteins, metabolites, and other molecular components may be necessary to achieve high sensitivity and specificity in diagnosing CKD and identifying individuals at high risk of progression. By combining multiple biomarkers, we can capture the various changes that occur throughout the course of the disease and obtain a more comprehensive understanding of CKD.

The present disclosure solves the technical problems in the art with the methods and systems for fluid retention analysis in Chronic Kidney Care, which discloses a comprehensive approach involving omics technologies, genetic markers, and a combination of biomarkers can help in creating a specific digital biomarker for CKD detection.

The methods and systems of the present disclosure propose an in-silico model-based approach which is used to fuel a Generative AI engine to generate physiological signals pertaining to CKD for various stages across demographics. The in-silico model is a computer-based model that uses a physics-based model such as a Monte Carlo forward modelling to simulate the Photoplethysmography (PPG) signal. This model can mimic the water content in parts of the body like the finger and how it changes with the development and spread of chronic kidney disease (CKD). The methods and systems of the present disclosure work in three stages. In the first stage, a standard PPG waveform is simulated using the physics-based model and compared it to reference experimental PPGs to make sure that the simulated PPG signals are accurate. In the second stage, a Generative Adversarial Network (GAN) model is trained using the simulated PPG data and the reference experimental PPG data. In the third and the last stage, the discriminator model of the trained GAN model is employed to evaluate and analyze the test dataset of the subject whose CKD is to be detected.

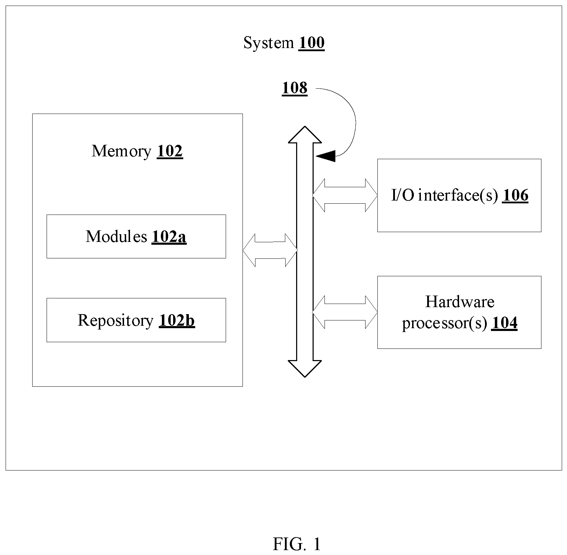

Referring now to the drawings, and more particularly to FIG. 1 through FIG. 4, where similar reference characters denote corresponding features consistently throughout the figures, there are shown preferred embodiments, and these embodiments are described in the context of the following exemplary system and/or method.

FIG. 1 is an exemplary block diagram of a system 100 for generating for fluid retention analysis in Chronic Kidney Care, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

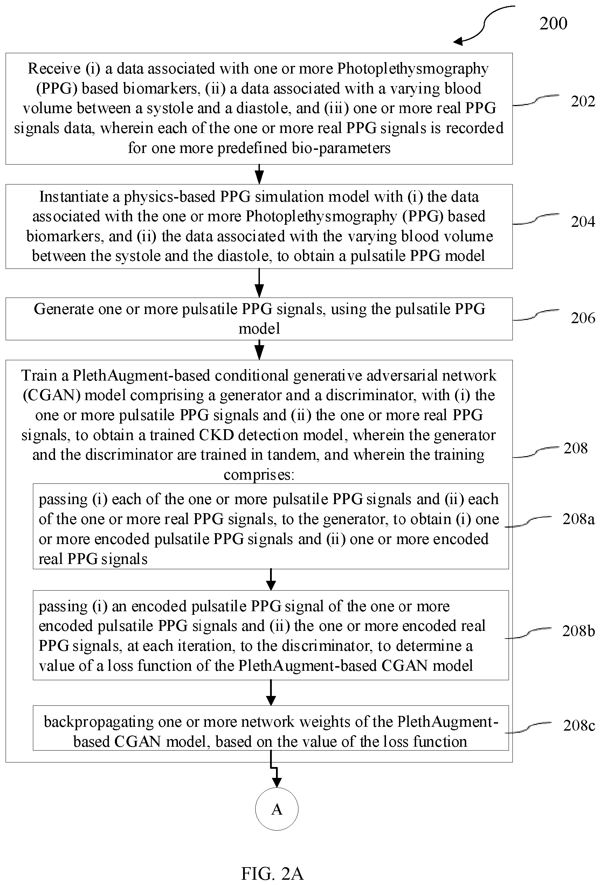

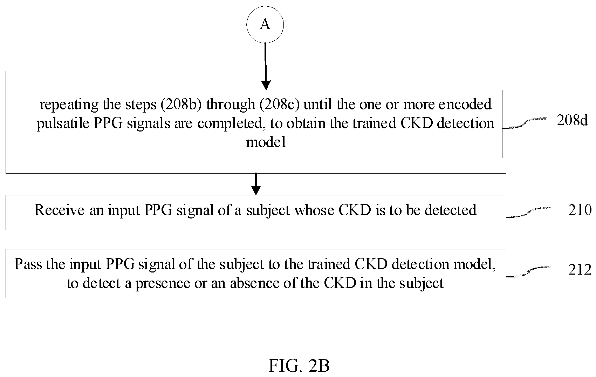

Referring to FIGS. 2A-2B, components and functionalities of the system 100 are described in accordance with an example embodiment of the present disclosure. For example, FIGS. 2A-2B illustrate exemplary flow diagrams of a processor-implemented method 200 for fluid retention analysis in Chronic Kidney Care, using the system of FIG. 1, in accordance with some embodiments of the present disclosure. Although the steps of the method 200 shown in FIGS. 2A-2B including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method 200, the one or more hardware processors 104 of the system 100 are configured to receive a data associated with one or more Photoplethysmography (PPG) based biomarkers, a data associated with a varying blood volume between a systole and a diastole, and one or more reference PPG signals. The one or more Photoplethysmography (PPG) based biomarkers refer to a combination of biomarkers that can help in creating a specific digital biomarker for CKD detection.

A comprehensive data-set that includes information about patients with CKD, including their medical history, clinical measurements, laboratory results, imaging data, and any other relevant information. This data-set will serve as the foundation for identifying the one or more potential biomarkers. In an embodiment, the one or more Photoplethysmography (PPG) based biomarkers include but are not limited to creatine, water content, and Beta-protein. The data associated with one or more Photoplethysmography (PPG) based biomarkers are the records of the biomarkers available from the PPG signals.

The data associated with the varying blood volume between the systole and the diastole refers to the variations in the input blood volume between the systole and the diastole. Here the input blood volume $V_b$ is considered such that the volume fraction of arterial and venous blood is equally distributed, i.e., $V_A$: $V_v$ = 1: 1. The one or more reference PPG signals are the real PPG signals that are recorded for one more predefined bio-parameters. In an embodiment, the one more predefined bio-parameters considered for recording the one or more reference PPG signals include but are not limited to age of the subjects, weight of the subjects, gender of the subjects, skin-tone of the subjects, fluid retention levels of the subjects, and body mass index (BMI) of the subjects. Here the subject refers to the living being including human beings and animals and including with and without CKD.

At step 204 of the method 200, the one or more hardware processors 104 of the system 100 are configured to instantiate a physics-based PPG simulation model with (i) the data associated with the one or more Photoplethysmography (PPG) based biomarkers, and (ii) the data associated with the varying blood volume between the systole and the diastole received at step 202 of the method 200. In the present disclosure, a Monte Carlo simulation model is used as the physics-based PPG simulation model. After the instantiation with the data associated with the one or more Photoplethysmography (PPG) based biomarkers, and (ii) the data associated with the varying blood volume between the systole and the diastole received, the physics-based PPG simulation model becomes a pulsatile PPG model which can be able to generate the dynamic PPG signals.

The physics-based PPG simulation model in general is a static light-tissue interaction-based PPG simulator. A traditional PPG sensor illuminates a tissue-section, usually, of a peripheral part of the body, by optical radiation, and the reflected/ transmitted light, is collected continuously along the cardiac cycles as the PPG signal. The physics-based PPG simulation model simulates the propagation of light through the heterogeneous tissue-section of the peripheral section of the body (e.g., fingertip) utilizing the forward Monte Carlo modeling strategy.

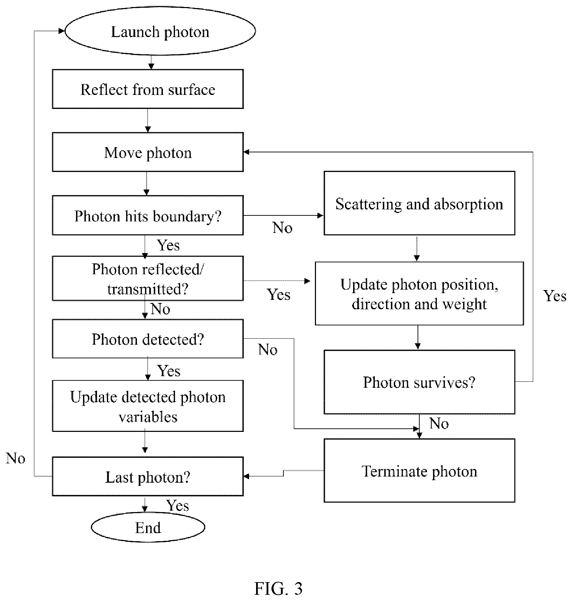

FIG. 3 shows an exemplary physics-based PPG simulation model, in accordance with some embodiments of the present disclosure. As shown in FIG. 3, the pathway of photons through tissue is determined by the geometrical configuration, i.e., how the sensor is placed on the tissue; and the anatomical configuration, i.e., the layer-stratification of the tissue-region of interest (ROI). In the present disclosure, a reflectance mode PPG sensor is assumed to be placed on the fingertip. The ROI is presented in a three-dimensional Cartesian co-ordinate plane, where the optical source of the sensor is placed at the origin of the co-ordinate system and the optical detector is placed as a distance d from the source. The detailed heterogeneous layer and sublayer stratification along with the various vascular distribution at different layers is considered.

In the given anatomical and geometrical configuration, the optical pathway through tissue is simulated by the random sampling of the probability of the interactions between light and tissue (e.g., absorption and scattering). The functional steps of the PPG simulator are:

Step 1: A photon packet with an initial direction and position co-ordinate and an initial statistical weight w = 1 is launched onto the tissue surface.

Step 2: After the initial correction for the reflection at the tissue surface, the photon packet is propagated through a step-size ($l$), calculated by random sampling of the probability of photon absorption and scattering coefficients ($\mu_a$ and $\mu_s$), i.e.,

$$l = \frac{\ln(\xi)}{\mu_a + \mu_s} \tag{1}$$

where $\xi$ is a computer-generated pseudo-random number ($0 < \xi < 1$).

Step 3: If the photon packet hits the boundary, a correction is made deciding whether it would reflect internally or transmit. If the photon transmits, it is checked whether it falls within the detection criteria. If the photon is detected, the remaining photon weight is scored, and the propagation of that photon packet is terminated.

Step 4: If the photon propagates freely, absorption and scattering events occur. A certain fraction of the photon-weight

(i.e., $\Delta w = \frac{\mu_a}{\mu_a + \mu_s} \times w^{'}$) is absorbed in the medium, and the rest of the weight continues to propagate. For the scattering, the direction of the photon packet is oriented through the randomly generated deflection and azimuthal angles. The scattering angle $\theta$ is calculated using the Henyey-Greenstein phase function whereas the azimuth is randomly generated between 0 and $2\pi$:

$$\theta = cos^{-1} \frac{1}{2g}\left[1 + g^2 - \left(\frac{1-g^2}{1-g+2g\xi}\right)\right] \tag{2}$$

$$\phi = 2\pi\xi$$

Step 5: The same steps are repeated until the incident photon packet is detected or discarded. The photon is discarded if the photon weight is too small or it transmits without being detected, and a new photon packet was launched. The process is repeated until a desired number of photon packets ($\approx 10^{10}$) are detected.

The Monte Carlo light-propagation through tissue depends on the absorption and scattering properties of the medium which vary significantly with the relative volume fractions of the tissue-constituents such as water, blood and other chromophores.

Considering the absorbance of light through arterial and venous blood with different concentrations of the absorbers oxyhaemoglobin ($HbO_2$) and deoxyhaemoglobin ($HHb$), the total absorption coefficient of any $i^{th}$ dermal sublayer can be written as:

$$\mu_{a_i}(\lambda) = V_{A_i}\mu_{a_{A_i}}(\lambda) + V_{V_i}\mu_{a_{V_i}}(\lambda) + V_{w_i}\mu_{a_{w_i}}(\lambda) + \left[1 - \left(V_{A_i} + V_{V_i} + V_{w_i}\right)\right]\mu_{a_{baseline_i}}(\lambda) \qquad (3)$$

where $V_A$ and $V_V$ stand for the arterial and venous blood volume-fraction respectively. $\mu_{a_A}$, $\mu_{a_V}$ and $\mu_{a_w}$ are the absorption coefficients of the arterial blood, venous blood and water. Considering $SaO_2$ and $SvO_2$ to be respectively the arterial and venous oxygen saturation, absorption coefficients of the arterial and venous blood can be written as:

$$\mu_{a_A}(\lambda) = SaO_2\mu_{a_{HbO_2}}(\lambda) + (1 - SaO_2)\mu_{a_{HHb}}(\lambda) \qquad (4)$$

$$\mu_{a_V}(\lambda) = SvO_2\mu_{a_{HbO_2}}(\lambda) + (1 - SvO_2)\mu_{a_{HHb}}(\lambda)$$

where $\mu_{a_{HbO_2}}$ and $g_{a_{HHb}}$ are the absorption coefficients of oxy and deoxyhaemoglobin, respectively.

The epidermal layer of the skin does not contain any amount of blood but only absorbs melanin and water. The melanin absorption coefficient $\mu_{a_{mel}}$ is determined from the following equation:

$$\mu_{a_{mel}}(\lambda) = 6.6 \times 10^{10} \times \mu_{a_A}(\lambda)^{-3.33} \qquad (5)$$

which is used to derive the absorption coefficient for epidermis:

$$\mu_{a_{epi}}(\lambda) = V_{mel}\mu_{a_{mel}}(\lambda) + V_{w_{epi}}\mu_{a_w}(\lambda) + \left[1 - \left(V_{mel} + V_{w_{epi}}\right)\right]\mu_{a_{baseline}}(\lambda) \qquad (6)$$

With the initiation and progression of CKD, water volume ($V_w$) changes significantly which is incorporated in the model, and the PPG wave is simulated for CKD stages. The optical properties of the tissue sample are defined by the equations relating to the volume distribution of tissue components (i.e., arterial, and venous blood, water, tissue layers etc.).

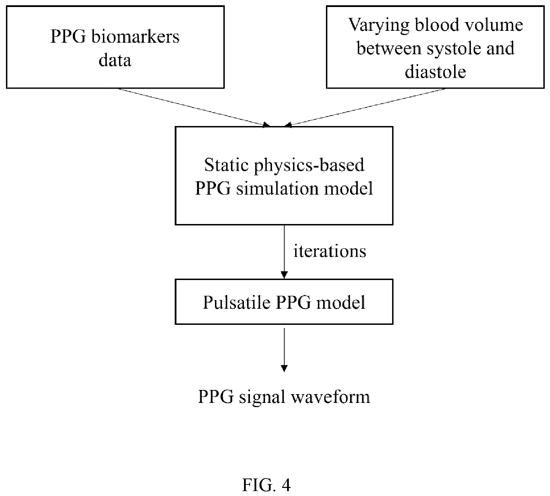

The equations (3) to (6) are the representative of the static PPG layer. For a pulsatile PPG model, these similar equations are used with the varying blood volume fractions. FIG. 4 is a flowchart showing the steps for obtaining the pulsatile PPG model by instantiating the physics-based PPG simulation model, in accordance with some embodiments of the present disclosure. As stated in FIG. 4, the input blood volume is varied between systole and diastole. For example, if the end systolic and end diastolic blood volumes are 50ml and 100ml, respectively, then the simulations are to be carried out for 50 blood volume fractions (V) between 50-100ml through a step of 50ml. Total blood volume being the combination of the arterial and venous blood volume $(V = V_A + V_V)$, the values are used in the equations (3) to (6) to obtain the specific optical properties of the model.

The physics-based PPG simulation model (the static PPG model) gives rise to the signal data for an instantaneous blood volume fraction. This model is iterated through the entire range if given blood volume fractions. Through iterations, the static PPG model, in turn, functions as the pulsatile PPG model. The pulsatile model gives rise to the PPG signal waveform for CKD.

At step 206 of the method 200, the one or more hardware processors 104 of the system 100 are configured to generate one or more pulsatile PPG signals, using the pulsatile PPG model obtained at step 204 of the method 200. In an embodiment, the one or more pulsatile PPG signals are generated for the varying blood volume between the systole and the diastole using the pulsatile PPG model and using the equation (3) to equation (6). Each of the generated pulsatile PPG signals is of a fixed duration (time-series).

At step 208 of the method 200, the one or more hardware processors 104 of the system 100 are configured to train a PlethAugment-based conditional generative adversarial network (CGAN) model, with the training dataset, to obtain a trained CKD detection model. The training data set comprises (i) the one or more pulsatile PPG signals generated at step 206 of the method 200 and (ii) the one or more reference PPG signals received at step 202 of the method 200.

The PlethAugment-based conditional generative adversarial network (CGAN) model comprises a generator and a discriminator. The generator and the discriminator are trained in tandem and compete against each other to create more authentic data from the training dataset. The generator produces synthetic data (from pulsatile PPG signals) that attempts to imitate reference data (reference PPG signals), while the discriminator separates the pulsatile PPG signals and the reference PPG signals.

In an embodiment, the generator is an auto-encoder network, and the discriminator is a time-series Convolutional Neural Network-Long Short-Term Memory (CNN-LSTM) model. The training process of the PlethAugment-based CGAN model is explained in detail through steps 208a to 208d.

At step 208a, each of the one or more pulsatile PPG signals is passed to the generator at a time to obtain the associated encoded pulsatile PPG signal. Similarly, each of the one or more reference PPG signals is passed to the generator to obtain the associated encoded reference PPG signal.

At step 208b, each encoded pulsatile PPG signal of the one or more encoded pulsatile PPG signals and (ii) the one or more encoded reference PPG signals, are passed at each iteration, to the discriminator, to determine a value of a loss function of the PlethAugment-based CGAN model. The loss function of the PlethAugment-based CGAN model is a sum of a generator loss and a discriminator loss. The generator loss is defined as a function of reconstruction loss, and the discriminator loss is defined as function of Binary Cross-Entropy loss.

At step 208c, one or more network weights of the PlethAugment-based CGAN model, are backpropagated based on the value of the loss function. Finally at step 208d, the steps (208b) through (208c) are repeated until the one or more encoded pulsatile PPG signals are completed, to obtain the trained CKD detection model. The trained CKD detection model is able to detect the presence or absence of the CKD in the subject.

At step 210 of the method 200, the one or more hardware processors 104 of the system 100 are configured to receive an input PPG signal of the subject whose CKD is to be detected.

At step 212 of the method 200, the one or more hardware processors 104 of the system 100 are configured to pass the input PPG signal of the subject received at step 210 of the method 200 to the trained CKD detection model obtained at step 208 of the method 200, to detect a presence or an absence of the CKD in the subject. More specifically, the discriminator of the trained CKD detection model tries to discriminate the input PPG signal of the subject and the one or more reference PPG signals used during the training to detect the presence or an absence of the CKD.

The methods and systems of the present disclosure discloses the comprehensive approach involving omics technologies, genetic markers, and the combination of biomarkers can help in creating the specific digital biomarker for CKD detection. The in-silico model-based approach is used to fuel a Generative AI engine to generate physiological signals pertaining to CKD for various stages across demographics. The in-silico model is a computer-based model that uses a physics-based model such as a Monte Carlo forward modelling to simulate the Photoplethysmography (PPG) signal. This model can mimic the water content in parts of the body like the finger and how it changes with the development and spread of chronic kidney disease (CKD). Hence the methods and systems of the present disclosure accurately and efficiently detect the presence or the absence of the CKD which is further used for effective fluid retention analysis in Chronic Kidney Care.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems of detecting the presence of CKD in the given subject based on the combinations of biomarkers effectively and accurately. The methods and systems of the present disclosure work in three stages. In the first stage, a standard PPG waveform is simulated using the physics-based model and compared it to reference experimental PPGs to make sure that the simulated PPG signals are accurate. In the second stage, a Generative Adversarial Network (GAN) model is trained using the simulated PPG data and the reference experimental PPG data. In the third and the last stage, the discriminator model of the trained GAN model is employed to evaluate and analyze the test dataset of the subject whose CKD is to be detected.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor-implemented method (200), comprising:

   receiving, via one or more hardware processors, (i) a data associated with one or more Photoplethysmography (PPG) based biomarkers, (ii) a data associated with a varying blood volume between a systole and a diastole, and (iii) one or more reference PPG signals, wherein each of the one or more reference PPG signals is recorded for one more predefined bio-parameters (202);

   instantiating, via the one or more hardware processors, a physics-based PPG simulation model with (i) the data associated with the one or more Photoplethysmography (PPG) based biomarkers, and (ii) the data associated with the varying blood volume between the systole and the diastole, to obtain a pulsatile PPG model (204);

   generating, via the one or more hardware processors, one or more pulsatile PPG signals, using the pulsatile PPG model (206); and

   training, via the one or more hardware processors, a PlethAugment-based conditional generative adversarial network (CGAN) model comprising a generator and a discriminator, with (i) the one or more pulsatile PPG signals and (ii) the one or more reference PPG signals, to obtain a trained CKD detection model, wherein the generator and the discriminator are trained in tandem (208), and wherein the training comprises:

      (a) passing (i) each of the one or more pulsatile PPG signals and (ii) each of the one or more reference PPG signals, to the generator, to obtain (i) one or more encoded pulsatile PPG signals and (ii) one or more encoded reference PPG signals (208a);

      (b) passing (i) an encoded pulsatile PPG signal of the one or more encoded pulsatile PPG signals and (ii) the one or more encoded reference PPG signals, at each iteration, to the discriminator, to determine a value of a loss function of the PlethAugment-based CGAN model (208b);

      (c) backpropagating one or more network weights of the PlethAugment-based CGAN model, based on the value of the loss function (208c); and

      (d) repeating the steps (b) through (c) for each of the one or more encoded pulsatile PPG signals, to obtain the trained CKD detection model (208d).

2. The processor-implemented method (200) as claimed in claim 1, further comprising:

receiving, via the one or more hardware processors, an input PPG signal of a subject whose CKD is to be detected (210); and

passing, via the one or more hardware processors, the input PPG signal of the subject to the trained CKD detection model, to detect a presence or an absence of the CKD in the subject (212).

3. The processor-implemented method (200) as claimed in claim 1, wherein the generator is an auto-encoder, and the discriminator is a time-series Convolutional Neural Network-Long Short-Term Memory (CNN-LSTM) model.

4. The processor-implemented method (200) as claimed in claim 1, wherein the loss function of the PlethAugment-based CGAN model is a sum of a generator loss and a discriminator loss, and wherein the generator loss is defined as a function of reconstruction loss, and the discriminator loss is defined as function of Binary Cross-Entropy loss.

5. A system (100), comprising:

a memory (102) storing instructions;

one or more input/output (I/O) interfaces (106); and

one or more hardware processors (104) coupled to the memory (102) via the one or more I/O interfaces (106), wherein the one or more hardware processors (104) are configured by the instructions to:

receive (i) a data associated with one or more Photoplethysmography (PPG) based biomarkers, (ii) a data associated with a varying blood volume between a systole and a diastole, and (iii) one or more reference PPG signals data, wherein each of the one or more reference PPG signals is recorded for one more predefined bio-parameters;

instantiate a physics-based PPG simulation model with (i) the data associated with the one or more Photoplethysmography (PPG) based biomarkers, and (ii) the data associated with the varying blood volume between the systole and the diastole, to obtain a pulsatile PPG model;

generate one or more pulsatile PPG signals, using the pulsatile PPG model; and

train a PlethAugment-based conditional generative adversarial network (CGAN) model comprising a generator and a discriminator, with (i) the one or more pulsatile PPG signals and (ii) the one or more reference PPG signals, to obtain a trained CKD detection model, wherein the generator and the discriminator are trained in tandem, and wherein the training comprises:

(a) passing (i) each of the one or more pulsatile PPG signals and (ii) each of the one or more reference PPG signals, to the generator, to obtain (i) one or more encoded pulsatile PPG signals and (ii) one or more encoded reference PPG signals;

(b) passing (i) an encoded pulsatile PPG signal of the one or more encoded pulsatile PPG signals and (ii) the one or more encoded reference PPG signals, at each iteration, to the discriminator, to determine a value of a loss function of the PlethAugment-based CGAN model;

(c) backpropagating one or more network weights of the PlethAugment-based CGAN model, based on the value of the loss function; and

(d) repeating the steps (b) through (c) for each of the one or more encoded pulsatile PPG signals, to obtain the trained CKD detection model.

6. The system (100) as claimed in claim 5, wherein the one or more hardware processors (104) are further configured by the instructions to:

receive an input PPG signal of a subject whose CKD is to be detected; and

pass the input PPG signal of the subject to the trained CKD detection model, to detect a presence or an absence of the CKD in the subject.

7. The system (100) as claimed in claim 5, wherein the generator is an auto-encoder, and the discriminator is a time-series Convolutional Neural Network-Long Short-Term Memory (CNN-LSTM) model.

8. The system (100) as claimed in claim 5, wherein the loss function of the PlethAugment-based CGAN model is a sum of a generator loss and a discriminator loss, and wherein the generator loss is defined as a function of reconstruction loss, and the discriminator loss is defined as function of Binary Cross-Entropy loss.

9. One or more non-transitory machine-readable information storage mediums comprising one or more instructions which when executed by one or more hardware processors cause:

receiving, (i) a data associated with one or more Photoplethysmography (PPG) based biomarkers, (ii) a data associated with a varying blood volume between a systole and a diastole, and (iii) one or more reference PPG signals, wherein each of the one or more reference PPG signals is recorded for one more predefined bio-parameters;

instantiating a physics-based PPG simulation model with (i) the data associated with the one or more Photoplethysmography (PPG) based biomarkers, and (ii) the data associated with the varying blood volume between the systole and the diastole, to obtain a pulsatile PPG model;

generating one or more pulsatile PPG signals, using the pulsatile PPG model; and

training a PlethAugment-based conditional generative adversarial network (CGAN) model comprising a generator and a discriminator, with (i) the one or more pulsatile PPG signals and (ii) the one or more reference PPG signals, to obtain a trained CKD detection model, wherein the generator and the discriminator are trained in tandem, and wherein the training comprises:

(a) passing (i) each of the one or more pulsatile PPG signals and (ii) each of the one or more reference PPG signals, to the generator, to obtain (i) one or more encoded pulsatile PPG signals and (ii) one or more encoded reference PPG signals;

(b) passing (i) an encoded pulsatile PPG signal of the one or more encoded pulsatile PPG signals and (ii) the one or more encoded reference PPG signals, at each iteration, to the discriminator, to determine a value of a loss function of the PlethAugment-based CGAN model;

(c) backpropagating one or more network weights of the PlethAugment-based CGAN model, based on the value of the loss function; and

(d) repeating the steps (b) through (c) for each of the one or more encoded pulsatile PPG signals, to obtain the trained CKD detection model.

10. The one or more non-transitory machine-readable information storage mediums of claim 9, wherein the one or more instructions which when executed by the one or more hardware processors further cause:

receiving an input PPG signal of a subject whose CKD is to be detected; and

passing the input PPG signal of the subject to the trained CKD detection model, to detect a presence or an absence of the CKD in the subject.

11. The one or more non-transitory machine-readable information storage mediums of claim 9, wherein the generator is an auto-encoder, and the discriminator is a time-series Convolutional Neural Network-Long Short-Term Memory (CNN-LSTM) model.

12. The one or more non-transitory machine-readable information storage mediums of claim 9, wherein the loss function of the PlethAugment-based CGAN model is a sum of a generator loss and a discriminator loss, and wherein the generator loss is defined as a function of reconstruction loss, and the discriminator loss is defined as function of Binary Cross-Entropy loss.

System **100**

**108**

Memory **102**

Modules **102a**

Repository **102b**

I/O interface(s) **106**

Hardware processor(s) **104**

FIG. 1

200

Receive (i) a data associated with one or more Photoplethysmography (PPG) based biomarkers, (ii) a data associated with a varying blood volume between a systole and a diastole, and (iii) one or more real PPG signals data, wherein each of the one or more real PPG signals is recorded for one more predefined bio-parameters — 202

Instantiate a physics-based PPG simulation model with (i) the data associated with the one or more Photoplethysmography (PPG) based biomarkers, and (ii) the data associated with the varying blood volume between the systole and the diastole, to obtain a pulsatile PPG model — 204

Generate one or more pulsatile PPG signals, using the pulsatile PPG model — 206

Train a PlethAugment-based conditional generative adversarial network (CGAN) model comprising a generator and a discriminator, with (i) the one or more pulsatile PPG signals and (ii) the one or more real PPG signals, to obtain a trained CKD detection model, wherein the generator and the discriminator are trained in tandem, and wherein the training comprises: — 208

passing (i) each of the one or more pulsatile PPG signals and (ii) each of the one or more real PPG signals, to the generator, to obtain (i) one or more encoded pulsatile PPG signals and (ii) one or more encoded real PPG signals — 208a

passing (i) an encoded pulsatile PPG signal of the one or more encoded pulsatile PPG signals and (ii) the one or more encoded real PPG signals, at each iteration, to the discriminator, to determine a value of a loss function of the PlethAugment-based CGAN model — 208b

backpropagating one or more network weights of the PlethAugment-based CGAN model, based on the value of the loss function — 208c

A

FIG. 2A

A

repeating the steps (208b) through (208c) until the one or more encoded pulsatile PPG signals are completed, to obtain the trained CKD detection model — 208d

Receive an input PPG signal of a subject whose CKD is to be detected — 210

Pass the input PPG signal of the subject to the trained CKD detection model, to detect a presence or an absence of the CKD in the subject — 212

FIG. 2B

Launch photon

Reflect from surface

Move photon

Photon hits boundary?

No → Scattering and absorption

Yes

Photon reflected/ transmitted?

Yes → Update photon position, direction and weight

No

Photon detected?

No

Yes

Update detected photon variables

Photon survives?

Yes

No

Update detected photon variables

Terminate photon

Last photon?

No

Yes

End

FIG. 3

PPG biomarkers data

Varying blood volume between systole and diastole

Static physics-based PPG simulation model

iterations

Pulsatile PPG model

PPG signal waveform

FIG. 4

Europäisches
Patentamt

European
Patent Office

Office européen
des brevets

## EUROPEAN SEARCH REPORT

**Application Number**

EP 25 16 4011

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SRIDEVI PARAMA ET AL: "Towards Developing a Mobile-Based Care (KidneyCare) for Patients with Kidney Diseases Using Ten-second Fingertip Video and PPG with Machine Learning", 2023 IEEE INTERNATIONAL CONFERENCE ON DIGITAL HEALTH (ICDH), IEEE, 2 July 2023 (2023-07-02), pages 202-204, XP034410851, DOI: 10.1109/ICDH60066.2023.00036 [retrieved on 2023-08-28] | 1,5,9 | INV. G16H50/20 A61B5/00 |
| A | * the whole document * | 2-4,6-8, 10-12 | |
| X | Sridevi Parama ET AL: "Title:-"Noninvasive Creatinine Estimation from PPG Signal Using ML Models with Optuna"", , 25 January 2024 (2024-01-25), XP093300604, Retrieved from the Internet: URL:https://papers.ssrn.com/sol3/papers.cfm?abstract_id=4694259 | 1,5,9 | |
| | | | TECHNICAL FIELDS SEARCHED (IPC) |
| A | * the whole document * | 2-4,6-8, 10-12 | G16H A61B |
| A | BHASKAR NAVANEETH ET AL: "Time Series Classification-Based Correlational Neural Network With Bidirectional LSTM for Automated Detection of Kidney Disease", IEEE SENSORS JOURNAL, IEEE, vol. 21, no. 4, 5 October 2020 (2020-10-05), pages 4811-4818, XP011832637, ISSN: 1530-437X, DOI: 10.1109/JSEN.2020.3028738 [retrieved on 2021-01-18] * the whole document * | 1-12 | |

-/--

The present search report has been drawn up for all claims

| Place of search | Date of completion of the search | Examiner |
|---|---|---|
| Munich | 31 July 2025 | Díaz de Lezana, C |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 16 4011

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHATTERJEE SUBHASRI ET AL: "Monte Carlo Analysis of Optical Interactions in Reflectance and Transmittance Finger Photoplethysmography", SENSORS, vol. 19, no. 4, 15 February 2019 (2019-02-15), page 789, XP093301101, CH ISSN: 1424-8220, DOI: 10.3390/s19040789 * the whole document * ----- | 1-12 | |

TECHNICAL FIELDS
SEARCHED (IPC)

The present search report has been drawn up for all claims

| Place of search | Date of completion of the search | Examiner |
|---|---|---|
| Munich | 31 July 2025 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 621 801 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421020145